**Europäisches Patentamt**

**European Patent Office**

**Office européen des brevets**

(19)

(11) Veröffentlichungsnummer: **0 111 027**
**A1**

(12) # EUROPÄISCHE PATENTANMELDUNG

(21) Anmeldenummer: **82111528.4**

(22) Anmeldetag: **13.12.82**

(51) Int. Cl.³: **G 01 N 33/76**
**G 01 N 33/78, G 01 N 33/50**

(43) Veröffentlichungstag der Anmeldung:
**20.06.84 Patentblatt 84/25**

(84) Benannte Vertragsstaaten:
**AT BE CH DE FR GB IT LI LU NL SE**

(71) Anmelder: **Steins, Knut**
**Mont-Cenisstrasse 26**
**D-4690 Herne 1(DE)**

(72) Erfinder: **Steins, Knut**
**Mont-Cenisstrasse 26**
**D-4690 Herne 1(DE)**

(74) Vertreter: **Herrmann-Trentepohl, Werner, Dipl.-Ing.**
**Schaeferstrasse 18**
**D-4690 Herne 1(DE)**

(54) Verfahren zur Frühestbestimmung der Gravidität, insbesondere beim Menschen sowie zur Durchführung dieses Verfahrens besonders geeignete Testreagenz.

(57) Um einen sicheren Nachweis für eine Gravidität zu schaffen, der bereits nach erfolgter Nidation einsetzt, wird abgenommenem Blut des zu untersuchenden weiblichen Wesens eine Mischung aus Bromwasserstoff, Permangansäure, Salpetersäure und wässriger Citratlösung gelöst in physiologischer Kochsalzlösung als Testreagenz zugesetzt, wobei das Mengenverhältnis von abgenommenem Blut und Testreagenz 1:1 gewählt wird. Das Verfahren beruht auf der chemischen Agglutination der Proteine, wobei die an Proteine gebundene Hormone und Glykogeneinlagerungen sowie der erhöhte Tetra- und Trijodthyroninspiegel ein Frühestanzeichen für eine bestehende Gravidität sind. Vorzugsweise ist beim verwendeten Testreagenz in 90 Vol.-Teilen physiologischer Kochsalzlösung eine Mischung aus

2 Vol.-Teilen 2,8 %-iger HBr
2 Vol.-Teilen 2,8 %-iger $HMnO_4$
2 Vol.-Teilen 5 %-iger $HNO_3$
4 Vol.-Teilen 10 %-iger $C_3H_4(OH)(COOH)_3 + H_2O$

gelöst.

4000 Herne 1,
Scl.ae'erstraße 18
Postfach 1140
Pat.-Anw. Herrmann-Trentepohl
Fernsprecher: 0 23 23 / 5 10 13
        5 10 14

Telegrammanschrift:
Bahrpatente Herne
T e l e x 08 229 853

Dipl.-Ing. R. H. Bahr (·· ····)    0111027
Dipl. - Phys. Eduard Betzler
Dipl.-Ing. W. Herrmann-Trentepohl
PATENTANWÄLTE
PROFESSIONAL REPRESENTATIVES
BEFORE THE EUROPEAN PATENT OFFICE

25 40 63
25 40 64
25 40 65

T e l e x 5 215 360
Telefax 089.79 89 88

Bankkonten:
Bayerische Vereinsbank München 952 287
BLZ 700 202 70
Dresdner Bank AG Herne 7-520 499
BLZ 432 800 84
Postscheckkonto Dortmund 558 68-457
BLZ 440 100 46

| Ref.: A 31 169 B/ag |
| In der Antwort bitte angeben |
| Zuschrift bitte nach: |
| **München** |

Ing. Knut Steins, Mont-Cenisstraße 26, 4690 Herne 1

---

Verfahren zur Frühestbestimmung der Gravidität, insbesondere beim Menschen sowie zur Durchführung dieses Verfahrens besonders geeignete Testreagenz

---

Die Erfindung betrifft ein Verfahren zur Frühestbestimmung der Gravidität insbesondere beim Menschen sowie eine zur Durchführung dieses Verfahrens besonders geeignete Testreagenz.

Im Humanbereich wurden in den letzten Jahren immer mehr Kinder mit Mißbildungen geboren. Sie stellen nicht nur die betroffenen Eltern, sondern auch die Ärzte und die Gesellschaft vor mannigfaltige Probleme.

Man unterscheidet bei den Mißbildungen zwischen

a) chromosomalen Störungen
b) monogenen Anomalien und Mißbildungen
c) Mißbildungen, bedingt durch ein Zusammenwirken von genetischen und exogenen Faktoren und

d) Mißbildungen, die nachweisbar nur durch exogene Faktoren verursacht werden.

Gerade bei den unter d) genannten Mißbildungen ist es wichtig, einen sicheren Reaktionstest zu schaffen, der frühzeitig eine Gravidität anzeigt, damit eben diese Schäden vermieden werden können. Unter derartigen exogenen Faktoren versteht man z.B.

1. die kurz- oder langfristige Einnahme bestimmter Medikamente,

2. Alkohol und/oder Zigarettenkonsum

3. diagnostizierende Maßnahmen, z.B. Kontrastmitteleinnahme, Röntgenstrahlen, radioaktive Einlagen, Narkosemittel.

Es existiert bisher kein Test, um eine Schwangerschaft vor Ausbleiben der Menstruation festzustellen. Die herkömmlichen Tests zur Feststellung einer Gravidität setzen erst ab 3. - 6. Woche nach der Konzeption ein und sind außerdem nicht sicher genug. In diesen Wochen ist aber die Entwicklung von Augen, Herz, Extremitäten, Zähnen, Ohren, Lippe, Gaumen und Bauch des Embryos bereits abgeschlossen.

Die heute existierenden Tests basieren zum Großteil auf dem Nachweis von HCG (Choriongonadotropin). Die Plazenta beginnt früh mit der Produktion verschiedener Hormone. Aber erst ca. 4 Wochen nach dem zur Empfängnis führenden Verkehr, also 10 bis 14 Tage nach der ausgebliebenen Menstruation reagieren die Tests. Es ist daher sinnlos einen derartigen Test früher durchzuführen. Mit den zur Zeit vorhandenen Methoden ist am frühesten der Anstieg des Gonadotropingehaltes des Blutes zu ermitteln. Die Ausscheidung der Gonadotropine erfolgt mit dem Urin, mit dem sie meist nachgewiesen werden.

Beim Mäusetest (Aschheim-Zondek-Reaktion: AZR) wird der Urin der zu untersuchenden Frau jugendlichen, weiblichen, weißen Mäusen eingespritzt. Nach 3-4-5 Tagen wird das innere Genital besichtigt. Enthielt der Urin Choriongonadotropin in ausreichender Menge (d.h. mehr als 7.000 bis 8.000 I.E.), so finden sich in den Ovarien blutgefüllter Follikel (Blutpunkte) und/oder Gelbkörper sowie vergrößerte Uterushörner. Beim Frosch- oder Krötentest (Galli-Manini-Test) wird erwachsenen männlichen Fröschen oder Kröten der zu untersuchende Urin in den dorsalen Lymphsack injiziert. Waren im Urin mehr als 10.000 I.E. HCG, so werden nach ca. 4 Stunden Spermien in die Kloake abgegeben.

Sowohl der Kaninchentest (Friedmann-Test) als auch der Rattentest (Frank-Berman-Test) entsprechen der Aschheim-Zondek-Reaktion. Das Testergebnis ist aber schon nach 1 bis 2 Tagen sichtbar.

Alle mit Tieren ausgeführten Tests werden als biologische Reaktionen zusammengefaßt. Der Nachteil all dieser biologischen Reaktionen liegt darin, daß die Testtiere jahreszeitlich schwankende Empfindlichkeit zeigen, so daß die bei ihnen auftretenden Reaktionen auch durch Produktion eigener Gonadotropine ausgelöst werden können.

Der immunologische Nachweis von Choriongonadotropin (Agglutationshemmtest) hat den Vorteil, daß diese Tests an keine Tierhaltung gebunden sind und durch Drogen, die die Testtiere zum Aussterben bringen können, nicht gestört werden. Andererseits bewirken aber eiweißhaltiger Urin und manche Medikamente einen falsch positiven Ausfall. Die immunologischen Tests können auf jede gewünschte Empfindlichkeit eingestellt werden. Üblicherweise sprechen sie auf 2.000 bis 5.000 I.E. HCG/l Urin an, werden also einige Tage früher eine Gravidität anzeigen als die Titertests. Bei noch größerer Empfindlichkeit drohen falsche positive Befunde, z.B. während des Klimakteriums.

Damit sich die HCG-Konzentration im Urin erhöht, ist es außerdem erforderlich, daß sich die zu untersuchende Patientin daran hält, ca. 12 Stunden vor dem Test möglichst wenig zu trinken.

Die Sicherheit der Labortests wird mit 90 - 95 % angegeben. Der Nachteil dieser Methode liegt darin, daß das HCG, nicht aber das Kind nachgewiesen wird.

Es gibt auch noch hormonale Schwangerschaftstests, die bei Frauen mit kurzdauernder Amenorrhoe durchgeführt werden. Man gibt ein Östrogen/Gestagen-Präparat, z.B. eine Ampulle Duogynon simplex i.m. oder je 1 Dragee Duogynon an zwei aufeinanderfolgenden Tagen. Falls nach 3 bis 6, spätestens nach 10 Tagen keine Blutung eintritt, ist mit einer Schwangerschaft zu rechnen.

Der Nachteil dieser Methode liegt darin, daß zur Sicherung der Diagnose spätere Untersuchungen notwendig werden. Darüber hinaus ist gerade das gebräuchlicherweise verwendete Östrogen/Gestagen-Präparat Duogynon in letzter Zeit wegen seiner möglichen schädigenden Wirkung auf den Foetus sehr stark umstritten.

Die Nachteile aller existenten Tests sind die, daß sie erstens nur im Humanbereich anwendbar sind, obwohl im Tierbereich der Anteil an Futter- und Haltungsschäden als exogene Schäden sehr hoch ist. Zweitens entstehen falsche positive Ergebnisse bei Medikamentenzugabe, auch während des Klimakteriums können falsche positive Befunde auftreten, oder auch Tumore können zu falschen Ergebnissen führen. Drittens ist der zeitliche Aufwand bei den Urintesten sehr hoch und der Nachweis des Gonadotropins im Blut oder Urin setzt zeitmäßig zu spät ein (erst ca. 4 Wochen nach dem zur Empfängnis führenden Verkehr), um eine Frühstschädigung in der frühen Blastogenese zu verhindern.

Daher ist es Aufgabe der vorliegenden Erfindung einen Test zu entwickeln, mit dem es möglich ist, Gravidität so frühzeitig zu erkennen, daß es möglich ist, entstehendes Leben auch bei ansonsten noch nicht erkennbaren Graviditäten vor exogenen Schäden zu schützen. Der Test soll dabei so wirksam sein, daß er sowohl im Human- als auch im Veterinärbereich (Säugetiere) anwendbar ist. Im Veterinärbereich soll insbesondere auch der Nachweis einer künstlichen Befruchtung so frühzeitig, d.h. im allgemeinen schon nach zwei Tagen erfolgen können, damit bei nicht erfolgter Befruchtung noch im gleichen Zyklus eine Insemination, d.h. eine erneute künstliche Befruchtung durchgeführt werden kann.

Ein in der Ampulle des Eileiters befruchtetes Ei braucht eine gewisse Zeit, um in den Uterus zu gelangen. Während dieser Zeit läuft die erste Entwicklung ab. Durch Enzymwirkung frißt sich das Ei in die Uterusschleimhaut ein (Nidation oder Implantation) und es kommt zu tiefgreifenden, wechselseitigen Beziehungen zwischen dem Uterus und dem Embryo.

Im Corpus luteum, dem nach der Ovulation aus dem Follikel durch Einlagerung von Carotinen entstandenen "Gelbkörpern" werden die Gestagene gebildet. Das wichtigste dieser Hormone ist das Progesteron.

Bei stattgefundener Konzeption bleiben die erhöhten Östrogen- und Progesteronspiegel erhalten. Ebenso fällt eine starke Glykogeneinlagerung in den basalen Bereichen der Epithelzellen, später auch in den Lumina der Drüsen auf. Dieses Glykogen ist an Proteine gebunden und bereits 24 bis 48 Stunden nach den Konzeption durch das Reagenz nach der Erfindung nachweisbar.

Im Hypophysenvorderlappen werden mehrere Hormone gebildet, welche ihrer chemischen Struktur nach Eiweiß- bzw. Polypeptidcharakter haben und durch Einwirkung auf periphere innersekretorische Drüsen den Hormonhaushalt des Organismus beeinflussen. Ein solches glandotropes Hormon ist zum Beispiel das ACTH (Adrenocorticotropes Hormon), das auf die Nebennierenrinde wirkt und deren Hormonproduktion so anregt, daß vermehrt Corticosteroide in die Blutbahn gelangen.

Es gibt zahlreiche Hormone, die auf das Geschehen in den Keimdrüsen einwirken, diese werden als gonadotrope Hormone bezeichnet. Sie sind nicht geschlechtsspezifisch. Man unterscheidet hauptsächlich zwei Wirkungen. Die Follikel-stimulierende Wirkung (FSH-Follikelstimulierendes Hormon) auf die Entwicklung der Follikel im Ovar und der Samenzellen im Hoden gerichtet und die Zwischenzellstimulierende Wirkung (ICSH = interstinal cell stimulating hormone), die die Hormonproduktion (Östron bzw. Testosteron) in den Zwischenzellen fördert und die identisch mit der luteinisierenden Wirkung (LH) ist. Das Thyreotrope Hormon (TSH) gehört zu den glandotropen Hormonen, es stimuliert die Schilddrüse. Es ist ein Glykoproteid bestehend aus zwei Ketten, wobei die $\alpha$-Kette mit der $\alpha$-Kette einiger Gonadotropine identisch zu sein scheint. Dieses Glykoproteid ist bereits 24 bis 48 Stunden nach der Konzeption feststellbar.

Andere Hypophysenvorderlappen (HVL)-Hormone, wie das Wachstumshormon (somatotropes Hormon = STH, neuerdings in der internationalen Literatur auch als growth hormone = GH bezeichnet) und der Exophtalamus produzierende Faktor (EPF) wirken direkt auf das Körpergewebe ein.

Das Luteotrope Hormon (LTH) oder Prolaktin wirkt gonadotrop, hat aber daneben direkten Einfluß auf den Kohlenhydratstoffwechsel.

Funktionell sind zumindest die meisten Hpophysenvorderlappenhormone den im Hypophtalamus gebildeten Wirkstoffen, den sogenannten "releasing Factoren" bzw. "-hormonen" (nach einem neuen Nomenklaturvorschlag auch als "Liberine" bezeichnet) untergeordnet.

Diese "hypophyseotropen" Hormone gelangen von ihrem Bildungsort im Hypophtalamus über ein Blutkappilarnetz des Hypophysenstiels und die sogenannten Portalgefäße der Adenohypophyse in den Hypophysenvorderlappen (der Mechanismus hierbei ist unterschiedlich zu dem Hypophtalamus-Hypophysenhinterlappensystem).

Längere Zeit bekannt ist das Thyreotropin-releasing-hormone (TRH), welches die TSH-Ausschüttung fördert. Chemisch besteht es aus drei Aminosäuren. Daneben kennt man ein Gonadotropin-releasing-hormone, das GNRH, bestehend aus 10 Aminosäuren mit Wirkung auf die HVL-Gonadotropine sowie einen Wachstumshormonreleasing-factor (GRF).

Während einer bestehenden Gravidität zeigen die Schilddrüsenhormone Thyroxin und Trijodthyronin zahlreiche fördernde und beschleunigende Einflüsse auf Wachstum, Reifung und Stoffwechselfunktionen des Organismus. Allerdings sind der Wirkungsmechanismus und der Angriffspunkt bis heute noch nicht genau bekannt. Die Höhe der Schilddrüsenhormonspiegel im Blut unterliegt folgendem Regelmechanismus:

```
  ┌ ─ ─ ─┤├ ─ →  ┌──────────────────┐
  │              │   Hypothalamus   │
  │              └──────────────────┘
  │                       │
  │                       │  TRH
  │                       ↓
  │    ┤├ →      ┌──────────────────┐
  ├───────────→  │  Hypophysen-     │
  │              │  vorderlappen    │
  │              └──────────────────┘
  │                       │
  │                       │  TSH
  │                       ↓
  │              ┌──────────────────┐
  │              │   Schilddrüse    │
  │              └──────────────────┘
  │                       │
  │                       │  Thyroxin, Trijodthyronin
  │                       ↓
  │       ┌──────────────────────────────┐
  └───────│  Blutspiegel an Schilddrüsen- │
          │  hormon und -metaboliten      │
          └──────────────────────────────┘
```

Wie aus dem vereinfachten Schema ersichtlich ist, besteht zwischen der Bildung und Ausschüttung der verschiedenen Hypophtalamus- und Hypophysenhormone einerseits und der Inkretproduktion der peripheren Drüsen bzw. direkten Stoffwechselwirkungen in den Geweben andererseits ein Regelmechanismus nach Art einer Rückkopplung. So führt eine Zunahme an freiem Hormon der peripheren Drüse im Blut meist über eine Beeinflussung des Hypophtalamus zu einer Hemmung der Ausschüttung an glandotropem Hormon.

Aus hypophtalamischen Zentren wird auf neurohumoralem Wege TRH (Thyreotropin-releasing-hormone) über den Hypophysenstiel in den Hypophysenvorderlappen abgegeben. Dort bewirkt TRH die Bildung und Ausschüttung von TSH (Thyroid-stimulating-hormone) ins Blut.

Zur Schilddrüsenhormonsynthese benötigt die Schilddrüse als essentiellen Bestandteil der Schilddrüsenhormone Jod. Aus dem Blut aufgenommenes Jodid wird
in der Schilddrüse gespeichert und zu elementarem
Jod oxidiert. Nach Jodierung von Thyrosin entstehen
durch Kondensation zweier Jodthyrosine die beiden
Schilddrüsenhormone Tetrajodthyronin (Thyroxin = T4)
oder Trijodthyronin (T3). Während ihrer Bildung und
Speicherung in der Schilddrüse sind T3 und T4 an
ein Glykoprotein gekoppelt (Thyreoglobulin).
Das normale Verhältnis von T4 zu T3 im Serum beträgt
etwa 70:1. Im Blut sind die Schilddrüsenhormone
größtenteils an Trägerproteine labil gebunden
(T4 zu ca. 99,9 %, T3 zu ca. 5 %).

Das PBJ (Protein-gebundenes Jod) beträgt normalerweise 3,5 - 7,0 mcg/100 ml Blut, bei Bestehen der
Gravidität verschiebt sich dieser Bereich auf 12 bis
22 mcg/100 ml Blut.

Die Aufgabe, einen sicheren Reaktionstest zu schaffen,
der bereits nach erfolgter Nidation einsetzt, um
frühzeitig eine Gravidität anzuzeigen, damit nachfolgende Schäden vermieden werden können, wird erfindungsgemäß dadurch gelöst, daß dem abgenommenem Blut
des zu untersuchenden weiblichen Wesens ein Testreagenz,
bestehend im wesentlichen aus einer Mischung von
Bromwasserstoff, Permangansäure, Salpetersäure und
Citratlösung gelöst in physiologischer Kochsalzlösung,
zugesetzt wird.

Dieses Verfahren fußt auf den erhöhten Hormonkonzentrationen und Glykogenanreicherungen, sowie der
Erhöhung des Tetrajodthyronin.(Thyroxin) und
Trijodthyroninspiegels im Blut des zu untersuchenden
weiblichen Wesens.

Das Ergebnis des erfindungsgemäßen Verfahrens zeigt sich darin, daß das Blut nicht graviditärer Objekte koaguliert und das Blut graviditärer Objekte nicht koaguliert, was auf das Abspalten der an Protein gebundenen Hormone und Glykogene sowie der Tetra- und Trijodthyronine zurückzuführen ist.

Vorzugsweise besteht die Mischung aus 90 Vol.-Teilen einer einer physiologischen Kochsalzlösung und

2 Vol.-Teilen 2,8 %-iger HBr  
2 Vol.-Teilen 2,8 %-iger $HMnO_4$  
2 Vol.-Teilen 5 %-iger $HNO_3$  
4 Vol.-Teilen 10 %-iger $C_3H_4(OH)(COOH)_3 + H_2O$.

Die Durchführung des Testes ist einfach. Man benötigt nur einen Tropfen Blut der zu untersuchenden Person und einen Tropfen dieses Reagenz. Nach inniger Vermischung dieser beiden Substanzen durch Verrühren und anschließendem Schwenken des Objektträgers wird ein einfach abzulesendes Ergebnis innerhalb von 20 Sekunden erzielt. Das Blut nicht graviditärer Objekte gerinnt und das Blut graviditärer Objekte gerinnt nicht.

Das Verfahren nach der Erfindung beruht somit auf der chemischen Agglutination der Proteine, wobei die an Proteine gebundenen Hormone und Glykogeneinlagerungen sowie der erhöhte Tetra- und Trijodthyroninspiegel ein Frühestanzeichen einer bestehenden Gravidität sind.

Patentansprüche

1. Verfahren zur Frühestbestimmung der Gravidität insbesondere beim Menschen, dadurch g e k e n n - z e i c h n e t , daß abgenommenem Blut des zu untersuchenden weiblichen Wesens eine Mischung aus Bromwasserstoff, Permangansäure, Salpetersäure und wässrigen Citratlösung gelöst in physiologischer Kochsalzlösung als Testreagenz zugesetzt wird.

2. Verfahren nach Anspruch 1, beruhend auf der chemischen Agglutination der Proteine, wobei die an Proteine gebundenen Hormone und Glykogeneinlagerungen sowie der erhöhte Tetra- und Trijodthyroninspiegel ein Frühestanzeichen für eine bestehende Gravidität sind.

3. Verfahren nach Anspruch 1, dadurch g e k e n n - z e i c h n e t , daß das Mengenverhältnis von abgenommenem Blut und Testreagenz 1:1 gewählt wird.

4. Testreagenz zur Durchführung des Verfahrens nach Anspruch 1, dadurch g e k e n n z e i c h n e t daß in 90 Vol.-Teilen physiologischer Kochsalzlösung eine Mischung aus

2 Vol.-Teilen 2,8 %-iger HBr
2 Vol.-Teilen 2,8 %-iger $HMnO_4$
2 Vol.-Teilen 5 %-iger $HNO_3$
4 Vol.-Teilen 10 %-iger $C_3H_4(OH)(COOH)_3 + H_2O$

gelöst ist.

# EUROPÄISCHER RECHERCHENBERICHT

Europäisches
Patentamt

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| Y | US-A-3 883 304  (C.H. HAMILTON) <br> * Insgesamt * | 1-4 | G 01 N   33/76 <br> G 01 N   33/78 <br> G 01 N   33/50 |
| Y | US-A-3 389 968  (J.M. MASEN) <br> * Insgesamt * | 1,2,4 | |
| Y | US-A-3 494 744  (G. ZBOROWSKI) <br> * Insgesamt * | 1,2,4 | |
| A | US-A-3 702 821  (A.A. FERNANDEZ) | | |
| A | DE-A-1 910 056  (G. KNAPP et al.) | | |

--- 

RECHERCHIERTE SACHGEBIETE (Int. Cl ³)

G 01 N

Der vorliegende Recherchenbericht wurde für alle Patentansprüche erstellt.

| Recherchenort <br> DEN HAAG | Abschlußdatum der Recherche <br> 08-08-1983 | Prüfer <br> GRIFFITH G. |
|---|---|---|

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82